# EUROPEAN PATENT SPECIFICATION

(11) **EP 2 563 329 B1**
(45) Date of publication and mention of the grant of the patent: **12.04.2017**
(21) Application number: 11716264.4
(22) Date of filing: 26.04.2011
(51) Int. Cl.: A61K 9/20, A61K 31/702

(54) **ORALLY DISINTEGRATING TABLET CONTAINING ACARBOSE**
IM MUND ZERFALLENDE TABLETTE MIT ACARBOSE
COMPRIMÉ À DÉSINTÉGRATION ORALE CONTENANT DE L'ACARBOSE

(30) Priority: 27.04.2010 EP 10161114
(43) Date of publication of application: 06.03.2013
(73) Proprietor: Bayer Intellectual Property GmbH, 40789 Monheim (DE)
(72) Inventor: SCHNEEWEIS, Axel, 10247 Berlin (DE); LAICH, Tobias, 51069 Köln (DE)
(74) Representative: BIP Patents
(86) International application number: PCT/EP2011/056587
(87) International publication number: WO 2011/134962

(56) References cited:
- EP-A1- 0 837 069
- WO-A1-99/37308
- WO-A2-2009/071219
- CN-A- 101 411 715
- Anonymous: "Acarbose" In: "European Pharmacopooeia 5.1", April 2005 (2005-04), Council of Europe, Strasbourg, XP055100760, pages 2873-2874,
- ROWE R C ET AL: "STARCH", 1 January 2006 (2006-01-01), HANDBOOK OF PHARMACEUTICAL EXCIPIENTS, PHARMACEUTICAL PRESS [U.A.], GB, PAGE(S) 725 - 726, XP003014998, ISBN: 978-1-58212-058-4
- NAKANO YOSHINORI ET AL: "Preparation and evaluation of unpleasant taste-masked pioglitazone orally disintegrating tablets", INTERNATIONAL JOURNAL OF PHARMACEUTICS, ELSEVIER BV, NL, vol. 446, no. 1, 16 February 2013 (2013-02-16), pages 160-165, XP029005026, ISSN: 0378-5173, DOI: 10.1016/J.IJPHARM.2013.02.019
- KIMURA SHIN-ICHIRO ET AL: "Effect of granule properties on rough mouth feel and palatability of orally disintegrating tablets", INTERNATIONAL JOURNAL OF PHARMACEUTICS, vol. 484, no. 1 , pages 156-162, XP029205552, ISSN: 0378-5173, DOI: 10.1016/J.IJPHARM.2015.02.023

## Description

It was an object of the present invention to provide an orally disintegrating tablet (ODT) for the glycosidase inhibitor acarbose. The object is achieved with an orally disintegrating tablet according to claim 1. In order to obtain the desired properties, the ingredients have to be precompacted with an insoluble lubricant and to be premixed with a water-insoluble carrier.

Optimal action of glycosidase inhibitors as antidiabetic is aided by as uniform a distribution as possible of the active ingredient in the ingested food. Such a uniform distribution can be achieved with the aid of an orally disintegrating tablet. The tablet and the active ingredient dissolves in the mouth, and the active ingredient is swallowed as a solution and comes, in the stomach, to the ingested food as a solution and can easily distribute therein.

WO2009/071219 describes a process for preparing an ODT containing 30% acarbose, comprising the steps of pre-compacting acarbose having an average particle size of 125-800 µm, mixing with at least 50% water-insoluble carriers, and tableting.

J The preparation of orally disintegrating tablets of the active ingredient acarbose is problematic, since the active ingredient results in very hard, slow-dissolving tablets owing to its physicochemical properties. A fast-dissolving orally disintegrating tablet can be obtained when a large portion (<50%) of water-insoluble carriers is introduced into the tablet. The mouthfeel of these tablets is, however, not satistactory, since the large proportion of insoluble excipients on the tongue is perceived as a rough foreign substance.

The development work for the present invention therefore concentrated on formulations having a low water-insoluble proportion.

By selecting suitable excipients and a suitable process (precompacting of acarbose), formulations were found which both feel pleasant in the mouth and release very rapidly.

The object was achieved by the formulations presented below and the associated process:
The formulation according to the invention is an orally disintegrating tablet containing
   a) 1-30% pre-compacted Acarbose having an average particle size of 100 to 800 µm and having a moisture content of between 0 and 5%,
   b) 40-90% water-soluble carrier, wherein the water-soluble carrier is selected from a group consisting of mannitol, isomalt, sorbitol, lactose and maltodextrin,
   c) 1-50% microcrystalline cellulose as water-insoluble carrier.

It has a disintegration time of less than 60 sec, preferably less than 45 sec, more preferably less than 30 sec, even more preferably less than 20 sec. The water-soluble carrier is selected from a group consisting of mannitol, isomalt, sorbitol, lactose and maltodextrin, optionally used in a mixture with binders. For the properties and rapid solubility, it is important that the overall moisture of the orally disintegrating tablet is between 0-8%, preferably between 1-5%. The tablets have an abrasion of below 1% and have a breaking strength which is between 20-50 N, preferably between 25-45 N. Before tableting, the acarbose is brought to an average particle size of 100 to 800 µm, preferably between 100-600 µm.

### Examples:

### Formulation 1

| | Amount [mg] |
|---|---|
| Constituents | |
| Acarbose | 50 000 |
| Ludiflash® | 111 100 |
| Microcrystalline cellulose | 67 650 |
| Crospovidone | 12 500 |
| Citric acid | 2500 |
| Apple aroma | 2500 |
| Green dye | 1250 |
| Magnesium stearate | 2500 |
| | |
| Weight | 250 000 |

### Formulation 2

| | Amount [mg] |
|---|---|
| Constituents | |
| Acarbose | 100 000 |
| Ludiflash® | 222 200 |
| Microcrystalline cellulose | 135 300 |
| Crospovidone | 25 000 |
| Citric acid | 5000 |
| Apple aroma | 5000 |
| Green dye | 2500 |
| Magnesium stearate | 5000 |
| | |
| Weight | 500 000 |

### Formulation 3

| | Amount [mg] |
|---|---|
| Constituents | |
| Acarbose | 50 000 |
| Ludiflash® | 111 100 |
| Microcrystalline cellulose | 67 650 |
| Crospovidone | 12 500 |
| Citric acid | 2500 |
| Apple aroma | 2500 |
| Green dye | 1250 |
| Sodium stearyl fumarate | 2500 |
| | |
| Weight | 250 000 |

### Formulation 4:

| | Amount [mg] |
|---|---|
| Constituents | |
| Acarbose | 100 000 |
| Ludiflash® | 222 200 |
| Microcrystalline cellulose | 135 300 |
| Crospovidone | 25 000 |
| Citric acid | 5000 |
| Apple aroma | 5000 |
| Green dye | 2500 |
| Sodium stearyl fumarate | 5000 |
| | |
| Weight | 500 000 |

### Formulation 5

| | Amount [mg] |
|---|---|
| Constituents | |
| Acarbose | 50 000 |
| Ludiflash® | 111 100 |
| Microcrystalline cellulose | 67 650 |
| Croscarmellose sodium | 12 500 |
| Citric acid | 2500 |
| Apple aroma | 2500 |
| Green dye | 1250 |
| Magnesium stearate | 2500 |
| | |
| Weight | 250 000 |

### Formulation 6:

| | Amount [mg] |
|---|---|
| Constituents | |
| Acarbose | 100 000 |
| Ludiflash® | 222 200 |
| Microcrystalline cellulose | 135 300 |
| Croscarmellose sodium | 25 000 |
| Citric acid | 5000 |
| Green dye | 5000 |
| Magnesium stearate | 5000 |
| | |
| Weight | 500 000 |

In the first step of the preparation, the acarbose is granulated with a lubricant; then the granulated substance is mixed with microcrystalline cellulose, such as Avicel for example. The granulation is achieved preferably by means of dry granulation. For this purpose, use is made of, for example, roller compactors, in which the powder is metered through a defined, narrow gap between two rotating rollers and is compressed by pressure alone to form flat, elongated strands, known as ribbons. These ribbons have to be reduced in size in a subsequent step so that they can be metered directly into a tablet press. The average particle size of the compact is between 100 and 800 µm, preferably between 100-600 µm. Most preferably, use is made of a compact having a particle size of at least 15% >250 µm.

After admixing further excipients, an orally disintegrating tablet containing 1-30% acarbose and 40-90% water-soluble carrier selected from a group consisting of mannitol, isomalt, sorbitol, lactose and maltodextrin, and 1-50% water-insoluble carrier which is microcrystalline cellulose is then prepared from

this compact by means of tableting. By precompacting the acarbose and subsequently admixing the components, the contact area between the acarbose and the excipients required for the disintegration is minimized. Therefore, the tablets prepared in this way have a disintegration time of less than 60 sec, preferably less than 45 sec, more preferably less than 30 sec, even more preferably less than 20 sec. The overall moisture of the orally disintegrating tablets is between 0 and 8%, preferably between 1 and 5%. The invention also relates to a process for preparing the claimed orally disintegrating tablets containing acarbose, comprising the steps
1) pre-compacting the Acarbose to get an average particle size of 100 to 800 µm
2) mixing with a water-insoluble carrier which is microcrystalline cellulose
3) mixing with a water-soluble carrier, selected from a group consisting of mannitol, isomalt, sorbitol, lactose and maltodextrin
4) tableting, characterized in that Acarbose having a moisture content of between 0 and 5% is used.

Optionally, point 2 and 3 can be combined.

Use is made of acarbose having a moisture content of between 0 and 5%, preferably between 1 and 4%. The tablets have an abrasion of below 1% and have a breaking strength which is between 10-50 N, preferably between 15-45 N. Most preferably, use is made of an acarbose compact having a particle size of 15% >250 µm.

It is common to all the formulations that the acarbose is not processed in a pure form with the water-soluble filler. Using a pure form leads to hard tablets. By enveloping with Avicel in an intermediate step, a rapid disintegration of the tablet is also achieved with the addition of a water-soluble filler. An advantage of the water-soluble filler is the better mouthfeel of the formulation, and also the better stability with respect to the disintegration time of the tablet. The tablets are characterized by the stability being at least 2 years, preferably 3 years.

Example: Determining the disintegration time of tablets comprising pure acarbose and precompacted acarbose

| | **Acarbose, pure** | **Acarbose, precompacted particles** |
|---|---|---|
| **Disintegration [s]** | | |
| Start | 13 s | 9 s |
| 6 weeks, 25° | 13 s | 7 s |
| 6 weeks, 40° | 41 s | 12 s |
| 12 weeks, 25° | 17 s | 11 s |
| 12 weeks, 40° | 43 s | 15 s |

## Claims

1. Orally disintegrating tablet containing
a) 1-30% pre-compacted Acarbose having an average particle size of 100 to 800 µm and having a moisture content of between 0 and 5%,
b) 40-90% water-soluble carrier, wherein the water-soluble carrier is selected from a group consisting of mannitol, isomalt, sorbitol, lactose and maltodextrin,
c) 1-50% microcrystalline cellulose as water-insoluble carrier.

2. Tablet according to Claim 1 containing pre-compacted Acarbose having a average particle size of 100 to 600 µm.

3. Tablet according to Claims 1 and 2 having a disintegration time of less than 60 sec.

4. Tablet according to Claims 1 and 2 having an overall moisture between 0 und 8%.

5. Process for preparing orally disintegrating tablets according to claims 1 to 4 containing Acarbose, comprising the steps
a) pre-compacting the Acarbose to get an average particle size of 100 to 800 µm
b) mixing with a water-insoluble carrier which is microcrystalline cellulose
c) mixing with a water-soluble carrier, selected from a group consisting of mannitol, isomalt, sorbitol, lactose and maltodextrin
d) tableting, **characterized in that** Acarbose having a moisture content of between 0 and 5% is used.

6. Process according to Claim 5, **characterized in that** acarbose having a mean particle size of 100 to 600 µm is used.

7. Orally disintegrating tablet according to Claims 1 to 4 for the treatment of diabetes mellitus.

## Patentansprüche

1. Im Mund zerfallende Tablette enthaltend
a) 1 - 30 % vorkompaktierte Acarbose mit einer durchschnittlichen Partikelgröße von 100 bis 800 µm und mit einem Feuchtegehalt zwischen 0 und 5 %,
b) 40 - 90 % wasserlöslichen Träger, wobei der wasserlösliche Träger ausgewählt ist aus einer Gruppe bestehend aus Mannit, Isomalt, Sorbit Lactose und Maltodextrin,
c) 1 - 50% mikrokristalline Cellulose als wasserunlöslichen Träger.

2. Tablette nach Anspruch 1, enthaltend vorkompaktierte Acarbose mit einer durchschnittlichen Partikelgröße von 100 bis 600 µm.

3. Tablette nach Ansprüchen 1 und 2 mit einer Zerfallszeit von weniger als 60 sec.

4. Tablette nach Ansprüchen 1 und 2 mit einer Gesamtfeuchte zwischen 0 und 8 %.

5. Verfahren zur Herstellung von schnell löslichen Tabletten nach Ansprüchen 1 bis 4 enthaltend Acarbose, umfassend die Schritte
a) Vorkompaktierung der Acarbose, um eine durchschnittliche Partikelgröße von 100 bis 800 µm zu erzielen,
b) Mischen mit einem wasserunlöslichen Träger, bei dem es sich um mikrokristalline Cellulose handelt,
c) Mischen mit einem wasserlöslichen Träger, ausgewählt aus einer Gruppe bestehend aus Mannit, Isomalt, Sorbit, Lactose und Maltodextrin,
d) Tablettierung, **dadurch gekennzeichnet, dass** Acarbose mit einem Feuchtegehalt von zwischen 0 und 5 % eingesetzt wird.

6. Verfahren nach Anspruch 5, **dadurch gekennzeichnet, dass** Acarbose mit einer durchschnittlichen Partikelgröße von 100 bis 600 µm eingesetzt wird.

7. Im Mund zerfallende Tablette gemäß den Ansprüchen 1 bis 4 zur Therapie von Diabetes mellitus.

## Revendications

1. Comprimé à désagrégation orale comportant
a) entre 1 et 30 % d'Arcarbose précompacté de granulométrie moyenne comprise entre 100 et 800 µm et de teneur en humidité comprise entre 0 et 5 %,
b) entre 40 et 90 % de vecteur hydrosoluble, où le vecteur hydrosoluble est choisi dans un groupe constitué par les suivants : mannitol, isomalt, sorbitol, lactose et maltodextrine,
c) entre 1 et 50 % de cellulose microcristalline en tant que vecteur insoluble dans l'eau.

2. Comprimé selon la Revendication 1, contenant de l'Arcarbose précompacté de granulométrie moyenne comprise entre 100 et 600 µm.

3. Comprimé selon les Revendications 1 et 2 de durée de délitement inférieure à 60 secondes.

4. Comprimé selon les Revendications 1 et 2 d'humidité globale comprise entre 0 et 8 %.

5. Procédé d'élaboration de comprimés à désagrégation orale selon les revendications 1 à 4 contenant de l'Acarbose, comprenant les étapes de
a) précompactage de l'Acarbose pour obtenir une granulométrie moyenne comprise entre 100 et 800 µm
b) mélangeage avec un vecteur insoluble dans l'eau qui est la cellulose microcristalline
c) mélangeage avec un vecteur hydrosoluble, choisi dans un groupe constitué par les suivants : mannitol, isomalt, sorbitol, lactose et maltodextrine
d) pastillage, **caractérisé en ce que** de l'Acarbose de teneur en humidité comprise entre 0 et 5 % est utilisé.

6. Procédé selon la Revendication 5, **caractérisé en ce que** de l'acarbose de granulométrie moyenne comprise entre 100 et 600 µm est utilisé.

7. Comprimé à délitement oral selon les Revendications 1 à 4 destiné au traitement du diabète sucré.
